# EUROPEAN PATENT SPECIFICATION

(11) **EP 1 281 716 B1**
(45) Date of publication and mention of the grant of the patent: **09.11.2005**
(21) Application number: 02015128.8
(22) Date of filing: 06.07.2002
(51) Int. Cl.: C07K 1/36, C07K 14/81, C12N 15/62

(54) **Purification process based on enzyme/tagged-peptide binding**
Reinigungsverfahren auf Grundlage der Bindung von Enzymen and markierte Peptide
Procédure de purification basée sur la liaison enzymes/peptides-marqués

(30) Priority: 10.07.2001 US 901996
(43) Date of publication of application: 05.02.2003
(73) Proprietor: Roche Diagnostics GmbH, 68305 Mannheim (DE); F.HOFFMANN-LA ROCHE AG, 4070 Basel (CH)
(72) Inventor: Dwulet, Francis Edward, Greenwood, Indiana 46142 (US); Balgobin, Neil Gwyn, Carmel, Indiana 46032 (US); McCarthy, Robert Crane, Carmel, Indiana 46033 (US)

(56) References cited:
- SECHI S ET AL: "A method to define the carboxyl terminal of proteins" ANALYTICAL CHEMISTRY, vol. 72, no. 14, 15 July 2000 (2000-07-15), pages 3374-3378, XP002223787 ISSN: 0003-2700
- LU Q ET AL: "Using Schizosaccharomyces pombe as a host for expression and purification of eukaryotic proteins" GENE, vol. 200, no. 1-2, 24 October 1997 (1997-10-24), pages 135-144, XP004126487 ISSN: 0378-1119

### SEQUENCE LISTING

<110> ROCHE DIAGNOSTICS GMBH
<120> ENZYME/TAG BINDING AND DETECTION SYSTEM
<130> 19308
<160> 13
<170> Patent In version 3.0
<210> 1
   <211> 10
   <212> PRT
   <213> mammalian
<220>
   <221> misc_feature
   <222> (4)..(4)
   <223> the amino acid at this position can be lysine or arginine
<220>
   <221> misc_feature
   <222> (5)..(5)
   <223> the amino acid at this position can be glycine or alanine
<220>
   <221> misc_feature
   <222> (6)..(6)
   <223> the amino acid at this position can be arginine, glycine or serin
<400> 1
   Gly Pro Cys Xaa Xaa Xaa Phe Ile Arg Tyr
   1               5                   10
<210> 2
   <211> 11
   <212> PRT
   <213> mammalian
<220>
   <221> misc_feature
   <222> (1)..(1)
   <223> the amino acid at this position can be asparagine or glycin e
<220>
   <221> misc_feature
   <222> (4)..(4)
   <223> the amino acid at this position can be proline or threonine
<220>
   <221> misc_feature
   <222> (5)..(5)
   <223> the amino acid at this position can be lysine or arginine
<220>
   <221> misc_feature
   <222> (8)..(8)
   <223> the amino acid at this position can be asparagine or aspart ate <400> 2
<210> 3
   <211> 9
   <212> PRT
   <213> snake venom
<220>
   <221> misc_feature
   <222> (2)..(2)
   <223> the amino acid at this position can be arginine or leucine <400> 3
<210> 4
   <211> 9
   <212> PRT
   <213> plant protease inhibitors
<220>
   <221> misc_feature
   <222> (1)..(1)
   <223> the amino acid at this position can be arginine or proline
<220>
   <221> misc_feature
   <222> (2)..(2)
   <223> the amino acid at this position can be leucine or proline
<220>
   <221> misc_feature
   <222> (4)..(4)
   <223> the amino acid at this position can be isoleucine or serine
<220>
   <221> misc feature
   <222> (5)..(5)
   <223> the amino acid at this position can be threonine or arginin e
<400> 4
   Xaa Xaa Arg Xaa Xaa Phe Ile Pro Asp
   1               5
<210> 5
   <211> 11
   <212> PRT
   <213> plant protease inhibitors
<220>
   <221> misc_feature
   <222> (5)..(5)
   <223> the amino acid at this position can be lysine or arginine
<400> 5
   Cys Ile Cys Thr Xaa Ser Ile Pro Pro Gln Cys
   1 5 10
<210> 6
   <211> 10
   <212> PRT
   <213> bird egg white trypsin inhibitors
<220>
   <221> mise_feature
   <222> (4)..(4)
   <223> the amino acid at this position can be lysine or arginine
<220>
   <221> misc_feature
   <222> (7)..(7)
   <223> the amino acid at this position can be serine or lysine
<400> 6
   Val Ala Cys Xaa Ile Leu Xaa Pro Val Cys
   1 5 10
<210> 7
   <211> 10
   <212> PRT
   <213> bovine basic pancreatic trypsin inhibitor <400> 7
<210> 8
   <211> 10
   <212> PRT
   <213> Soybean Kunitz protease inhibitor
<400> 8
   Ser Pro Tyr Arg Ile Arg Phe Ile Ala Glu
   1 5 10
<210> 9
   <211> 10
   <212> PRT
   <213> Soybean Bowman-Birk protease inhibitor
<400> 9
   Ala Ser Thr Lys Ser Asn Pro Pro Gln Ser
   1 5 10
<210> 10
   <211> 10
   <212> PRT
   <213> Sand Viper venom protease inhibitor
<400> 10
   Gly Arg Ser Lys Ala His Ile Pro Arg Phe
   1               5                   10
<210> 11
<211> 10
   <212> PRT
   <213> Bovine secretory protease
<400> 11
   Gly Ser Pro Arg Ile Tyr Asn Pro Val Ser
   1               5                   10
<210> 12
   <211> 10
   <212> PRT
   <213> Chicken ovomucoid domain 3 protease
<400> 12
   Val Ala Ser Arg Ile Leu Ser Pro Val Ser
   1               5                   10
<210> 13
   <211> 10
   <212> PRT
   <213> Chicken ovomucoid domain 4 protease
<400> 13
   Val Ala Ser Arg Ile Leu Leu Pro Val Ser
   1               5                   10

## Description

### FIELD OF THE INVENTION

The present invention relates to a process for the production and recovery of a protein molecule by recombinant DNA technology. An identification peptide and the desired protein are synthesized together as a fusion protein that can be purified or detected with the use of native or non-active capture proteins.

### SUMMARY OF THE INVENTION

The current invention describes a method of using a "capture" enzyme to bind a specific "tagged" peptide for purposes of affinity purification or detection. If native enzymes are used for these purposes, modification to the tagged protein can be eliminated by the use of tag sequences that bind but are not processed by the enzyme. Alternatively, the enzyme can be chemically or genetically modified so that one or more critical amino acid in the active site has been changed, thereby leading to loss of catalytic activity.

The use of enzymes as capture agents offers several advantages not currently found within the state of the art. For example, it is possible to select natural or recombinant capture proteins with modified binding sites that will have different affinities for the same tag. Conversely, it is possible to modify the amino acid sequence of the tag to generate high, medium and low affinity peptide tags for use in different applications with the same capture protein. Lastly, depending upon the application, natural or recombinant capture proteins with increased or decreased resistance to denaturation can also be prepared. Such versatility is amenable to the development of a variety of standardized binding and elution conditions for the isolation of tagged proteins or their complexes. This flexibility can be found in no other system available at this time.

### BACKGROUND OF THE INVENTION

Rapid affinity purification techniques utilizing immobilized capture agents that bind to peptide ligands (tags) biochemically incorporated into the genes of interest have become a common methodology used by molecular biologists to rapidly purify recombinant proteins. Advantages of these techniques are that they are universal (i.e., a peptide can easily be incorporated into any gene through recombinant technology) and easy to use (simple equipment: batch or gravity column, simple elution protocols).

There are many different systems currently in use to purify tagged proteins. The most popular system used today for such affinity purification is immobilized metal affinity chromatography (IMAC), wherein a repetitive sequence of histidine residues (usually 6) binds to a metal ion complex (usually nickel) that is attached to a resin. Examples of these systems are the QIAexpress™ system from Qiagen and the Talon™ system from Clontech. These resins are easy to use, have a high binding capacity, and can be regenerated for repetitive use. However, binding and elution from these supports have low selectivity and many non-tagged proteins co-purify with the tagged protein. In addition, the recovered protein contains high concentrations of imidazole and nickel salts. This method has one of the poorest purification efficiencies, which limits the utility of this method for purifying macromolecular complexes.

The second most commonly used systems are antibody-based methodologies such as the FLAG system from Sigma or the HA system from Roche Molecular Biochemicals. In these systems, monoclonal antibodies raised against peptide antigens are used to capture the specific tagged proteins. After washing to remove the untagged contaminants, the tagged proteins are recovered by the application of any of a number of specific and nonspecific elution agents. The selectivity of these systems is much better than the IMAC system described above. However, the antibodies used in such systems are very costly to manufacture and are easily denatured and inactivated when exposed to extremes of pH or chaotropic agents.

The next class of systems uses proteins as the tags and immobilized cofactors or organic ligands as the capture molecule. The two most used systems in this class are the Glutathione-S-transferase (GST) system of Amersham Pharmacia Biotech and the Thiofusion expression system of Invitrogen. The advantage of these systems is the low cost and durability of the capture agent. However, the large size of the tags (greater than 20,000 daltons molecular weight) often has a significant negative impact on the proper folding and functioning of the recombinant protein.

The last tag purification technology to be discussed is the carboxyl terminal lysine/arginine peptide purification system. Although not originally designed to purify tagged proteins, under the right conditions recombinant proteins can be created and purified. In this system, anhydrotrypsin (trypsin in which the active site serine residue has been chemically converted to dehydroalanine) is immobilized onto a support and used to capture peptides having carboxyl terminal lysine or arginine residues. Although this system uses one of the capture agents that we will describe later, the mechanisms of binding and release of the tagged proteins are totally different (see Table 1).

**TABLE 1:**

| Differences Between The Carboxyl Terminal Lysine/Arginine Purification Method And The Present Method. | | |
|---|---|---|
| Characteristics Lysine/Arginine Method Present Method | | |
| Type of tag sequence | Lysine/Arginine Peptides | Substrates/Inhibitors |
| pH binding profile | Maximal 5.5 to 6.5 | Maximal 7.0 to 8.0 |
| Elution with Benzoyl Arginine or Hippuryl Arginine | Yes | No |
| pH 3.0 elution | Yes | Yes |
| Internal Lysine or arginine peptide binding | No | Yes |
| Strength of Binding | Weak | Strong |
| Nature of interaction | Enzyme/Product | Enzyme/Substrate |
| Binding denatured samples | Yes | Yes |

In comparing the present method with the lysine/arginine method, it should be noted that the two properties these systems have in common are characteristics of the anhydrotrypsin molecule and not the tag. The literature that demonstrates the carboxyl terminal lysine and arginine peptide binding repeatedly teaches that internal (i.e., non-terminal) lysine and arginine sequences do not bind strongly to the anhydrotrypsin matrix. This view is probably a result of the focus of the studies (the recovery of carboxyl terminal lysine and arginine peptides) and not on characterizing the properties of the anhydrotrypsin molecule. Our findings on the binding and elution characteristics of native and denatured trypsin inhibitors to anhydrotrypsin were totally unexpected from the current literature and generally accepted theories.

All of the existing tagged affinity purification systems have one or more deficiencies. In some of the existing systems the binding resin can only be used for a few cycles of purification, for others the binding capacity of the resin is very low, while other systems are unable to purify proteins as part of a macromolecular complex. Some of the current systems have low purification efficiencies and in others the affinity resin is very expensive. In addition, some of the systems require very large tag sequences that can have a negative impact on the recombinant protein structure and function. The majority of the protein-based isolation systems employ capture proteins that are not resistant to denaturation and thus have limited utility in the purification of denatured or aggregated protein complexes. The apparent reason for this is that these proteins were chosen based on their unique binding characteristics and not on their resistance to denaturation.

In contrast, many of the enzymes of the present invention function in adverse environments, are resistant to denaturation, and therefore are ideally suited for use in this application. The invention described in this document uses small substrate or inhibitor polypeptide tags that bind to natural, modified, altered or mutated enzymes to purify tagged recombinant proteins. The diversity of the proteins that can be used for this invention and their general resistance to denaturation makes them ideal binding proteins for this application.

The present invention offers significant advantages over previously used affinity systems. In the present invention, the binding that is present between the enzyme and the target peptide sequence (usually a sequence derived from an enzyme substrate or inhibitor specific for the active site of the enzyme) can be selected to offer a wide range of binding constants. Thus, tags can be designed to have the desired binding and elution characteristics. Within the super family of all enzymes, the subfamily of proteolytic enzymes is especially well suited for our needs. Most of these proteins are small single chain molecules with molecular weights in the range of 15,000 to 30,000 daltons. They tend to have very compact structures and a propensity to refold back to their native structures. Lastly, proteolytic enzymes tend to be more resistant to denaturation, and thus are useful under conditions not suitable for many current affinity purification systems.

The present invention is based on the concept of using protein-protein interactions as the basis for affmity Purification In the most general case, the topography of one protein is complementary to that of the other and the two interact to form a non-covalent complex. This is the basic mechanism of antibody-antigen interactions. However, this type of protein-protein interaction is rather general, and can be found in many biological interactions. The activities of many enzymes are controlled by protein-protein interactions. One aspect of this invention, described herein, is a protease and protease substrate system where a protease is a protein that fragments other proteins or polypeptides and a protease substrate is a protein or polypeptide that specifically binds to the active site of the protease. Inhibitors are a special subset of these substrates. These polypeptide inhibitors can have a high affinity for the enzyme active site and through any of a number of mechanisms form a complex that is inactive. In this aspect of the invention, the enzyme (either active or chemically /genetically inactivated) is attached to a solid support to create an affinity matrix. Using molecular biology techniques, the peptide sequence that binds to the active site of the enzyme is cloned into the gene of interest and this genetic construct is expressed in an appropriate protein expression system. After expression, the tagged protein is recovered by passage of the crude material over a matrix containing the immobilized enzyme. The tagged recombinant protein will bind strongly to the immobilized enzyme while the other proteins will be washed away. The tagged protein is then recovered by more stringent elution conditions.

### DETAILED DESCRIPTION OF THE INVENTION

In describing the present invention, a "natural protein" is defined as a polypeptide with a defined amino acid sequence that is capable of being produced in a living cell. The gene coding for this protein will be found as a normal component of the DNA of a cell or an infecting bacteria or virus. The proteins will have one or more defined three-dimensional structures and have one or more defined or unknown functions. Our interest is with proteins that are enzymes that interact with other polypeptides that are substrates, co-factors or inhibitors. Isoenzymes and genetic polymorphisms are considered natural enzymes.

A "modified enzyme" is a protein in which one or more amino acid residues in the active site have been chemically or genetically modified to eliminate the enzyme's catalytic function while maintaining its ability to bind substrates, co-factors and inhibitors. These modifications include chemical modification of amino acid side chains as well as genetic substitution, insertion and deletion of amino acid residues.

An "altered enzyme" is a protein that has been chemically or genetically modified at a non-catalytic residue. These modifications include chemical modification of amino acid side chains as well as genetic substitutions, insertions and deletions of amino acid residues. These alterations can have a variety of effects, and can be neutral with minimal effect on affinity, specificity, activity or stability (neutral modification). These substitutions can affect the stability of the protein with respect to environmental factors. Some examples of these factors include temperature, pH, salt and denaturing agents. Other substitutions could have an effect on the catalytic site and lead to changes in affinity, specificity and activity.

In one embodiment, the present invention is a system used for affinity purification. This system comprises two elements primarily. The first element is the "capture reagent" and the second is the "tag."

The capture reagent is an enzyme that has a binding site for specific peptide sequences. The binding of the peptide sequence without adverse effects on the tagged protein can be accomplished by either modifying the peptide sequence (the "tag") that binds to the active site or by using a protein as a capture agent which is able to bind but not modify the tag.

The former option can be accomplished if the molecular mechanisms of catalysis and the active site topography are well understood. For example, a number of protease inhibitors are known to bind and inhibit active enzymes without suffering proteolysis, and therefore, these protease inhibitors are useful as tag sequences in the present invention.

For the latter option, there are a large number of proteins (enzymes) that could be used in their natural or modified form for this application. The first group that comes to mind are the proteases such as the serine, sulfhydryl and acidic proteases. All of these proteases only use their side chain amino acids to create their active sites. Using site-directed mutagenesis techniques, these enzymes could be modified to inactivate their active sites. For the serine proteases, such as trypsin or enterokinase, the active site serine residues can be replaced by alanine. In a similar way, the active site cysteine residue for the sulfhydryl-containing enzymes of papain or chymopapain could also be replaced with alanine orserine residues. Finally, acidic proteases like pepsin could have one or more of their active site aspartic acid groups replaced with a serine or asparagine residue.

For those systems using active enzyme as the capture agent the preparation of the solid phase absorbent is straight forward. Using any of a variety of solid phase supports, the active natural or recombinant enzyme is immobilized using a variety of linkage chemistries to prepare the capture matrix. Such immobilization procedures are well known in the art.

The only caveat to this process is that care must be taken not to immobilize the capture enzyme with an amino acid residue that is in or near the active site of the protein. If this occurs the capture molecule will have either reduced capacity or affinity for the tag. Bovine trypsin is known to lose activity upon immobilization unless an inhibitor like benzamidine is used to protect the active site. Such concerns are well known to the art when proteins are covalently linked to other molecules.

For embodiments using capture enzymes lacking proteolytic activity, the process is often a bit more complicated. As an illustration, the gene for the proenzyme trypsinogen can be modified using site-directed mutagenesis methods to change the active site serine residue at position 183 to alanine. Published data show that chemical modification of this residue does not distort the active site, and normal L-alanine is expected to allow for proper folding. In a similar way, the gene for enterokinase (more accurately renamed enteroprotease) light chain can have the active site serine residue at position 187 changed to an alanine residue. In either case, a genetic construct can be made containing the gene for the specific protein. The protein can then be expressed in an appropriate protein expression system. After synthesis, the modified recombinant trypsinogen or enterokinase can be recovered by any of a number of chromatographic techniques. If necessary, the capture protein can be enzymatically processed to its final form and immobilized to a number of different supports and used to isolate the specific tagged recombinant protein.

A natural protein that can be used as the capture molecule is human heparin binding protein (CAP 37/Azurocidin). This protein is a member of the serine protease family but the active site triad residues have been mutated to yield a protein with an elastase-like structure but with no enzymatic activity. However, this protein is still capable of binding strongly to trypsin inhibitors and as such would make an excellent capture molecule.

A large number of options are available to develop the tag peptide sequence. For methods of the present invention employing active enzyme capture agents, there are at least three strategies which can be used. Combinatorial chemistry or synthetic peptide synthesis techniques can be used to generate a large number of peptides. The relative binding strengths of each peptide to the binding protein and its resistance to modification is easily determined. Once sequences with the desired characteristics have been identified, they can be tested for their effectiveness to purify a tagged recombinant protein. This would be a "shotgun-type" (i.e., broadly focused) technique and could be applied to any enzyme.

A more focused approach would be to start with a sequence having a known affinity for the enzyme and then either using site directed mutagenesis or chemical synthesis to create analogs that could be evaluated for their characteristics. As an example, for proteases, one could use published data on the amino acid sequences of protease inhibitors. The basic pancreatic trypsin inhibitors are known to have very high binding constants and resistance to proteolysis. Detailed evaluation of homologs of these sequences can lead to the development of peptides that bind to the enzyme but are not cleaved. In another approach, one could just use the entire inhibitor molecule or a disulfide bridged binding domain of an inhibitor with the desired characteristics as the tag.

For methods of the present invention using capture enzymes which have been modified so that they can bind but not modify the tag, the same types of characterizations could be performed, but the only data of interest would be the binding affinity and release criteria for the tag sequences. In summary, the use of enzymes to isolate tagged recombinant proteins offers a valuable tool to meet the practical application of rapidly isolating recombinant proteins.

Once prepared, the present invention can also be used for the detection, localization or quantitation of the tagged proteins. Similar technologies have been extensively developed using monoclonal antibodies as the binding partner for the identification of tagged proteins. Like those technologies, the present system has all of the properties required for detecting, localizing or quantitating tagged proteins. In its simplest form, a reporter molecule is attached to the capture protein and the signal from this molecule is used to obtain the desired information. In general, any label or reporter molecule which can be incorporated into an antibody can be incorporated into our enzyme system. A representative but not exclusive list of labeling technologies includes direct labels such as spectrophotometric or fluorescent molecules as well as amplification reagents such as biotin or enzymes.

The following paragraphs will provide a more detailed discussion of the interactions of trypsin and trypsin inhibitors with their implications for a tagged peptide purification system. Although there are many different families of trypsin inhibitors, their mode of action is believed to follow the same basic mechanism. Trypsin inhibitors contain disulfide bonds and these bonds are considered essential for inhibitor function. Polypeptide trypsin inhibitors inhibit in a competitive manner. Trypsin inhibitor that has had its disulfide bonds oxidized loses its ability to inhibit trypsin. During the inhibition process a single peptide bond is usually proteolytically cleaved in the inhibitor binding sequence and both the native and cleaved inhibitor can bind to trypsin. These facts led to the currently accepted theory of the mechanism of trypsin and trypsin inhibitor interaction.

Under this accepted theory, the trypsin inhibitor disulfide bonds are critical to lock the inhibitor sequence into the proper conformation so it can properly fit into the trypsin active site in the typical "lock and key" model of enzyme-substrate interaction. The inhibitor is fragmented at the specific bond and the linked inhibitor chains separate slightly and catch themselves into the trypsin active site. This increases the binding energy of interaction, reducing the dissociation constant and blocking the active site of the trypsin. The interaction is non-covalent and dissociation and reassociation can occur. This theory is supported by enzymatic, x-ray crystallographic and physical biochemical findings and for the most part it seems to explain the interaction.

We determined that reduced, alkylated and denatured trypsin inhibitors bind to anhydrotrypsin with about the same affinity as the native inhibitor proteins. This is a significant and unexpected observation. These results are consistent with the conclusion that the primary structure of the trypsin inhibitor and the topography of the trypsin active site are the determining factors for the binding constant of the interaction. In addition, it appears that the function of the disulfide bonds is to hold the proteolyzed peptides of the trypsin inhibitor together and thus reduce the dissociation constant. Even when the proteolyzed inhibitor dissociates from the enzyme it can still reassociate with the enzyme and inhibit its activity. In the inhibitor without the disulfide bridges, once proteolysis occurs, the peptides can individually dissociate from the enzyme. Once separate in solution they are not able to interact with the trypsin as an inhibitor, and instead become simply another substrate. This then strongly shows that modified proteins like anhydrotrypsin can be used as a capture agent and that trypsin inhibitor active site sequences without disulfide bridges can be used as tags.

In higher animals, especially within the pancreas, there exist at least two different families of protease (trypsin) inhibitors. These inhibitors are generally classified as the Kunitz and secretory families of inhibitors. The Kunitz inhibitors are generally intracellular. They form very strong 1:1 complexes with trypsin that have dissociation constants reported to be as low as 10⁻¹⁴ moles/liter. This is a very low dissociation constant and is within the dissociation range of the strongest non-covalent interactions. This inhibitor is usually not proteolyzed by the enzyme. Upon binding, these inhibitors permanently inactivate the enzyme. These proteins are about 60 amino acid residues long with three disulfide bridges. The inhibitor sequence is found on the second disulfide loop with the amino acids which bind to the cleavage site (lysine or arginine) being on the carboxyl side of the cysteine residue. The general sequences of these inhibitor sequences are as follows:

-C- X-Y-Y-Z-Z-

wherein
C = Cysteine
X = Lysine or Arginine
Y = small or polar amino acid
Z = Hydrophobic or aromatic amino acid (Formula I).

The consensus sequence for this class of inhibitor is as follows.

The secretory trypsin inhibitors in the pancreas are secreted into the pancreatic duct along with the zymogen proteases. Upon binding, these inhibitors generally prevent the premature activation of the enzymes. These inhibitors have a much weaker binding constant than the Kunitz inhibitors and the active bond of the inhibitor is usually fragmented during the inactivation process. These inhibitors will eventually be degraded by the trypsin. These proteins have a structure similar to the Kunitz inhibitors. They are also about 60 amino acid residues long and contain three similar disulfide bridges. The inhibitor sequence is also found in the second disulfide loop. The structure in this loop is similar to the Kunitz sequences, and is represented by the following:

-C-A-X-Z-Z-Y-B -Z-

wherein
C = Cysteine
A = variable amino acids (usually threonine or proline)
X = Lysine or arginine
Y = small or polar amino acid (usually asparagine or aspartic acid)
B = Hydrophobic (usually proline)
Z = Hydrophobic or aromatic amino acids (Formula II).

The consensus sequence for these proteins is as follows:

The snake venom protease inhibitors are variations of the Kunitz pattern. They tend to be small proteins of about 60 amino acids with three disulfide loops. They have active site sequences with the following pattern:

-C-X-Y-B-Z-Z-

wherein
C = Cysteine
X = Lysine or Arginine
Y = small amino acid (usually alanine)
B = variable amino acid (usually polar)
Z = Usually hydrophobic (Formula III)

The consensus sequence for these proteins is as follows:

Plants, like animals, have a number of families of protease inhibitors. The two most common are the Kunitz and the Bowman-Birk families. The Kunitz family is a high affinity protease inhibitor that is about 190 amino acid residues long with two disulfide bridges. The inhibitory site is located in the middle of the first large loop. The overall inhibitor site has the following general structure:

-B-A-X-Y-Y-Z-Z-

wherein
X = Usually Arginine
A = Usually hydrophobic
Y = Variable usually small (alanine and threonine) or polar
Z = Usually hydrophobic (isoleucine, leucine, phenylalanine or tyros ine)
B = Variable (proline or arginine most common) (Formula IV).

The consensus sequence is more variable:

The Bowman-Birk inhibitor family is similar to the animal secretory family of inhibitors. The proteins are small (70 to 80 amino acid residues) with around seven disulfide bridges. The inhibitors are double-headed with both trypsin and chymotrypsin inhibitory sites. The inhibitor sequences are on very tight disulfide loops. The dissociation constants for the trypsin-inhibitor complexes is between 10⁻⁸ to 10⁻¹⁰ M. The general sequence is as follows:

-C-A-X-A-Y-Z-Z-B-C-

wherein
C = cysteine
A = polar amino acid (usually threonine or serine)
X = usually lysine
Y = variable amino acid (usually isoleucine)
Z = hydrophobic amino acid (usually proline)
B = polar amino acid (Formula V)

The consensus Bowman-Birk Inhibitor sequence is as follows:

The last family of inhibitors is the bird egg white trypsin inhibitors. Bird egg protease inhibitors are commonly referred to as "ovomucoids." These proteins are some of the more heavily glycosylated proteins found in egg white. They are members of the Kazal family of protease inhibitors, and exhibit a very high homology to the secretory inhibitors of other animals. The general sequence is as follows:

-C-A-X-Z-Z-Y-B -Z-

wherein
C = Cysteine
A = variable amino acids (usually proline)
X = Lysine or arginine
Y = small or polar amino acid (usually serine or lysine)
B = Hydrophobic (usually proline)
Z = Hydrophobic or aromatic amino acids (Formula VI).

The consensus sequence for these proteins is as follows.

Several things should be noted in conjunction with this information:
a) From X-ray crystallographic data the amino acids on the amino terminal side of the active lysine or arginine residue do not significantly contact with the active site of the trypsin. Therefore, only the Lys or Arg residue along with the following five or six amino acid residues will bind into the active site.
b) In general all of the inhibitor sequences have a relatively similar distribution of residues in their sequence. Following the lysine or arginine residue there will be two amino acids that are usually small or polar. Basic amino acids are often seen in the second of these two positions. The next two positions are almost always hydrophobic. Only the secretory inhibitors do not follow this pattern but their function is different from the other proteins.
c) Also, our reduction alkylation experiments demonstrated that the cysteine residues do not contribute significantly to the binding and so for these sequences this amino acid residue could be replaced.

There are several possible inhibitor tag sequences that constitute specific embodiments of the "tag" sequence useful in the present invention. respectively).

When used as a tag, these sequences (like any of the tag sequences of the present invention) can contain greater or fewer amino acids than those listed above, so long as the binding activity with the capture enzyme remains sufficiently strong.

With regards to these inhibitors, there appears to be at least two mechanisms of inhibition. With the secretory inhibitors there are two hydrophobic amino acid residues (usually leucine and isoleucine) on the carboxyl side of the active lysine or arginine. It appears that this hydrophobic region protects the sensitive amide linkage by increasing the energy of activation for the formation of the tetrahedral intermediate. This would then slow down the kinetics of hydrolysis. It is well known from protein sequence studies that the presence of multiple hydrophobic residues on the carboxyl side of lysine and arginine residues greatly reduces the kinetics of proteolytic cleavage.

The Kunitz inhibitors seem to utilize a different mechanism for inhibiting the enzyme. These inhibitors have amino acid sequences, located at the carboxyl side of the sensitive bond, that have a very high binding constant to the enzyme. It seems that once binding occurs, these sequences have a very low rate of dissociation. Thus, these structures seem to inhibit the enzyme by increasing the energy of activation for the dissociation of the product-enzyme complex. The venom inhibitors seem to be a variation on the theme of the Kunitz inhibitors.

There are several means of eluting the tagged protein from the immobilized capture agent. We have demonstrated that lowering the pH to a value of about 3.0 is sufficient to elute the tagged protein. This is a standard method for the dissociation of trypsin inhibitor from trypsin or anhydrotrypsin. These conditions are known to cause a mild reversable denaturation of the trypsin active site. An elution buffer that we have found to be very effective is 50 mM glycine, 10 mM calcium chloride pH 3.0. Another method of elution useful in the present invention is to use native trypsin inhibitor as the elution agent to compete for the binding site of the enzyme. Bovine basic pancreatic trypsin inhibitor (BPTI) is an example of a native inhibitor that is useful for elution. It is a small peptide (about 70 AA long) and has a very high binding affinity for trypsin. Depending on the specific tag that is used, it is possible to use many to most of the different trypsin inhibitors. This elution reagent could be used at any pH but has its strongest binding at values between 7.0 to 8.5.

Another alternative is to use a synthetic peptide from the inhibitory sequence. This would require a seven to eight residue peptide that would be small enough for easy removal. Again elution conditions would be similar to those for the native inhibitor.

Another possibility would be to use low molecular weight competitive inhibitors such as benzamidine. This would have an even lower molecular weight and would probably be the least costly. Because these inhibitors have a lower affinity than the natural peptide inhibitors, higher molar concentrations would be required.

It should be noted that the binding of the trypsin inhibitor sequences used in the present invention to the anhydrotrypsin has a very different mechanism of binding and elution from that of polypeptides containing carboxyl terminal lysine and arginine residues (see Table 1). First, the optimal binding pH values are different with the present inhibitor sequences binding stronger at neutral to slightly alkaline (7 - 8.5) and the carboxyl terminal lysine and arginine peptides binding stronger at slightly acidic values (5.5 - 6.5). Finally the carboxyl terminal peptides can be eluted from the anhydrotrypsin by low molecular weight arginine peptides (hippuryl arginine and benzoly arginine) while the present inhibitor sequences are not. The binding and elution characteristics of these structures are totally different and seem to follow distinct mechanistic pathways.

While we have studied the protease-protease inhibitor system in great detail, it is not the only possible example for this system. There are many additional enzymes that could be used as capture molecules. Two additional enzyme families that could be used as capture molecules are kinases and glycosylating enzymes. The kinases are a large family of proteins whose function is to place a phosphate group on specific serine, threonine or tyrosine residues. Some of these enzymes only recognize very specific sequences and could perform as very high stringency capture agents. This family of proteins can be divided into a number of sub families of enzymes based upon the sugar chain that will be covalently linked and the specific amino acid being modified. One of the best characterized of these enzymes is the asparagine - high mannose glycosylating enzymes. These proteins transfer a preformed high mannose carbohydrate structure to specific asparagine residues. These enzymes only recognize asparagine residues in specific exposed sequences which have the structure Asn-Xaa-(Ser/Thr)-, where Xaa can be any amino acid so long as the sequence is sufficiently solvent exposed to fit into the enzyme active site. Modification of the carbohydrate binding site or the residues responsible for the transfer of the carbohydrate chain onto the asparagine residue could convert this enzyme into a capture protein. These families of enzymes are presented as examples of the types of proteins which can be used as capture agents and sequences which can be used as tags. Individuals familiar with the art can identify many more examples of enzymes that can be used as capture agents and polypeptide substrates or inhibitors that could be used as tags.

The present invention provides an efficient process for the recovery of recombinant proteins using an identification peptide having a high affinity for an enzyme capture protein. The tag can be a natural or synthetic polypeptide substrate, inhibitor, cofactor or effector amino acid sequence. The capture molecule is an enzyme (protein) that has a binding site for the tag sequence. This capture molecule may be a native protein (or fragment) or it may be modified (genetically or chemically) so as to still be able to bind the tag sequence but not modify it. A large number of embodiments of this concept are possible and several will be described below.

### EXAMPLES

### Example 1

One embodiment of this invention employs a modified trypsin as the capture protein and the active sequences of trypsin inhibitors as the tags. The trypsin molecule (either natural or recombinant) has an active site amino acid modified so that the protein is still capable of binding the tag (inhibitor) but is not capable of proteolytic fragmentation. Although a number of residues in the active site of trypsin could be modified, the most effective and easiest to modify is the serine residue that is responsible for peptide bond hydrolysis (in bovine trypsin this is residue 177). A number of publications have reported methods to accomplish this modification but the method of Ishii et al. (Methods in Enzymology 91: 378 - 383) was found to be the most effective.

The protocol for the preparation of the modified trypsin is briefly described as follows. Trypsin is first reacted with phenylmethylsulfonyl chloride (PMSF) to form the sulfonate ester on the active site serine hydroxyl group. This esterified trypsin is then treated with alkali to induce a beta elimination of the modified serine residue with the formation of a dehydroalanine residue. The dehydrated trypsin (anhydrotrypsin) is then purified on an arginine affinity resin. The final product is a protein with the active site of trypsin but without the ability to fragment peptide bonds. The anhydrotrypsin can be attached to a variety of solid supports and used as an affinity matrix. Within the literature a number of these supports have been prepared and their ability to bind carboxyl terminal lys ine and arginine peptides has been characterized and is well-known in the art.

We have evaluated the binding and release of native and denatured trypsin inhibitors to the immobilized anhydrotrypsin as a model to characterize the purification of tagged proteins. First we determined the pH profile of binding of the trypsin inhibitors to the anhydrotrypsin solid support. What was observed was a profile that matched the activity vs. pH plot of native trypsin with maximal binding being observed between pH values of 7.0 to 8.0. This is in contrast to the carboxyl terminal lysine or arginine peptides that have a pH maximum of binding around pH 5.0 to 5.5. Also, the carboxyl terminal lysine and arginine peptides can be eluted by organic derivatives of arginine while these reagents do not elute trypsin inhibitor sequences. The denaturation of the trypsin inhibitors with urea and guanidine hydrocholoride shows little loss of binding capacity and this seems follow the denaturation of trypsin by these reagents. Lastly, completely denatured, reduced and carboxymethylated trypsin inhibitors were found to bind at least as well as the native proteins to the anhydrotrypsin in buffers with and without urea or guanidine. This binding of the denatured trypsin inhibitors to the anhydrotrypsin support provides strong evidence that the specific binding of these proteins is driven by the primary sequence of the inhibitor binding region and not by its conformation and presentation to the trypsin molecule.

Characteristics of Anhydrotrypsin-Trypsin Inhibitor Tag Purification System The immobilized Anhydrotrypsin is a single chain disulfide bridged protein with very high stability. Samples of this resin were repeatedly exposed to denaturing buffers containing up to 6 molar guanidine hydrochloride or 8 molar urea with no loss of binding capacity. Also, binding capacity is unaffected by repeated exposure to acidic conditions (pH 2.5 to 3.0). Samples of the resin have been kept at +2 to 8°C for over six months without loss of binding capacity.

Binding of trypsin inhibitor sequences to anhydrotrypsin occurs outside of the pH optimum for carboxyl terminal arginine and lysine peptides and proteins that have arginine and sine residues at their carboxyl terminal. Thus, use of such pH ranges allows a reduction of nonspecific binding of these peptides. Further, nonspecifically bound proteins can be eluted with arginine containing buffers. It has also been determined that bound proteins can be recovered by mild acidification of the support. In conclusion this technology has all of the characteristics for an optimal system.

A simple alternative for the chemical modification of native trypsin would be to clone the gene for trypsin and replace the active site serine residue with another amino acid. Although many amino acid residues might fit into the active site, structural and steric considerations indicate that an alanine residue is a preferred choice for this substitution.

An alternative to using trypsin would be to use chymotrypsin. Chymotrypsin is very homologous to trypsin, enzymatically operates using the same mechanism and there are many chymotrypsin inhibitor sequences available.

### Example 2

An additional embodiment of this invention would be to use a modified enzyme with a restricted substrate specificity as the capture molecule and the substrate sequence as the tag. Several examples of possible capture enzymes are enterokinase or factor Xa. Both of these enzymes are serine proteases with high homologies to trypsin and chymotrypsin. All of these enzymes use the same active site residues to fragment their substrate molecule.

Enterokinase is an intestine mucosal enzyme that recognizes the amino terminal sequence of trypsinogen and cleaves it to generate active trypsin. The specificity of this enzyme is very high and it appears to only bind and cleave proteins with this sequence. The key segment of this sequence is four aspartic acid residues followed by a lysine residue. Enterokinase cleaves the peptide bond at the carboxyl side of the lysine residue. In one embodiment, the active site serine residue can be modified as reported above for trypsin and chymotrypsin.

Enterokinase can be isolated from intestinal mucosa or may be produced synthetically or by recombinant techniques. In one embodiment, the active site serine (residue 187 of the mature protein) is converted to an alanine residue to yield a protein that can bind its substrate but not proteolytically fragment the molecule. Other substitutions within the active site are possible but from our investigations and that of others it seems that the serine residue has the least influence on substrate (inhibitor) binding. An interesting observation is that most plasmids that include a restriction protease cleavage site use the enterokinase sequence to allow for tag removal. Thus, for this system, there would be no need for the preparation of new vectors for the expression of the tagged recombinant proteins.

Another example of enzyme-substrate combinations useful in the present invention is factor Xa and its cleavage peptide. Factor Xa is also a serine protease and one of the enzymes in the clotting pathway. Factor Xa, like enterokinase, has a very stringent sequence requirement and will only cleave a peptide bond after the arginine of the sequence isoleucine - glutamic acid - glycine - arginine. As with the examples above, the active site serine residue of factor Xa can be modified by either chemical or genetic means to yield a capture protein without the ability to fragment the polypeptide chain. The factor Xa cleavage site is the second most popular cleavage site to be genetically engineered into vectors for cloning and so there are a large number of tagged proteins for which this capture agent would be of value.

### Example 3

An additional preferred embodiment of this concept would be to use a modified psychrophilic enzyme as the capture protein. Psychrophilic enzymes are proteins adapted to function at temperatures approaching 0°C and are usually found in non-warm blooded organisms in the Arctic and Antarctic. The ability to function at low temperatures comes at a cost. These proteins have reduced thermal stability. The trypsin isolated from North Atlantic cod has been shown to thermally denature at 15 °C lower temperature than bovine trypsin and the trypsin from the Antarctic fish Paranotothenia magellanica is inactivated at temperatures above 30°C. The genes for these proteins have been cloned and sequenced. These proteins show high homology to other fish and mammalian trypsins with unique cold adaptations. Like the capture enzymes described in example 1, these enzymes could be chemically modified or their genes could be modified to convert the active site serine residue to an alanine or other inert amino acid. These proteins would be immobilized and used as capture agents as described in example 1. The only difference is that elution could be performed by just raising the temperature. From a production and handling standpoint there would be significant advantages to such a system.

The present invention has been detailed both by direct description and by example. Equivalents and modifications of the present invention, e.g., using a modified psychrophilic enzyme as tag peptide sequence binding enzyme, will be apparent to those skilled in the art, and are encompassed within the scope of the invention.

## Claims

1. A method for purifying or isolating a recombinant fusion peptide, said method comprising the steps of:
a) forming a fusion peptide comprising a tag peptide sequence covalently attached to a polypeptide sequence;
b) contacting the fusion peptide with an enzyme or modified enzyme that specifically binds to the tag peptide sequence to form a complex between the enzyme or modified enzyme and the fusion peptide;
c) eluting non-complexed peptides to separate non-complexed peptides from the complexed peptide; and
d) conducting a second elution wherein the fusion peptide is dissociated from the enzyme or modified enzyme;
wherein said method does not require that the tag sequence have a lysine or arginine at its carboxyl terminal to maintain binding.

2. The method of claim 1 wherein the tag sequence is a substrate or inhibitor of the enzyme.

3. The method of claim 1 wherein the enzyme or modified enzyme is linked to a solid substrate.

4. The method of claim 1 wherein the tag sequence binds but is not cleaved by the enzyme.

5. The method of claim 1 wherein a modified enzyme is used, and the modified enzyme is a modified protease wherein the peptide sequence for the active site of said protease has been altered such that it binds, but does not cleave, the tag sequence.

6. The method of claim 5 wherein the modified enzyme is a serine protease in which the active site serine residue has been replaced with a nonactive amino acid such as alanine.

7. The method of claim 1 wherein the capture protein is a modified enterokinase.

8. The method of claim 1 wherein the modified enzyme is a modified psychrophilic enzyme and binding of the tag sequences to the capture protein occurs below the denaturation temperature of the enzyme and elution is performed by raising the temperature to near or above the denaturation temperature of the enzyme.

9. A method for purifying or isolating a recombinant fusion peptide, said method comprising the steps of:
a) forming a fusion peptide comprising a tag peptide sequence covalently attached to a polypeptide sequence, said tag peptide sequence containing a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11; SEQ ID NO: 12 and SEQ ID NO: 13;
b) contacting the fusion peptide with an enzyme or modified enzyme that specifically binds to the tag peptide sequence to form a complex between the enzyme or modified enzyme and the fusion peptide;
c) eluting non-complexed peptides to separate non-complexed peptides from the complexed peptide; and
d) conducting a second elution wherein the fusion peptide is dissociated from the enzyme or modified enzyme;
wherein said method does not require that the tag sequence have a lysine or arginine at its carboxyl terminal to maintain binding.

10. A method for tagging a recombinant peptide, said method comprising the step of forming a fusion peptide comprising a tag peptide sequence covalently attached to a polypeptide sequence, said tag peptide sequence containing a peptide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13.

## Patentansprüche

1. Ein Verfahren zur Reinigung oder Isolierung eines rekombinanten Fusionspeptids, wobei das genannte Verfahren die folgenden Schritte umfasst:
a) Bildung eines Fusionspeptids, umfassend eine Tag-Peptidsequenz, die kovalent an eine Polypeptidsequenz gebunden ist;
b) Kontaktieren des Fusionspeptids mit einem Enzym oder einem modifizierten Enzym, welches spezifisch an die Tag-Peptidsequenz bindet, um einen Komplex zwischen dem Enzym oder dem modifizierten Enzym und dem Fusionspeptid zu bilden;
c) Eluieren von nicht-komplexierten Peptiden, um die nicht-komplexierten Peptide von den komplexierten Peptiden zu trennen; und
d) Durchführen einer zweiten Eluation in der das Fusionspeptid von dem Enzym oder dem modifizierten Enzym dissoziert wird,
wobei das genannte Verfahren nicht verlangt, dass die Tag-Sequenz ein Lysin oder Arginin an ihrem Carboxyl-Terminus aufweist, um die Bindung aufrecht zu erhalten.

2. Verfahren gemäß Anspruch 1, bei dem die Tag-Sequenz ein Substrat oder Inhibitor des Enzyms ist.

3. Verfahren gemäß Anspruch 1, bei dem das Enzym oder das modifizierte Enzym an ein festes Substrat gebunden ist.

4. Verfahren gemäß Anspruch 1, bei dem die Tag-Sequenz an das Enzym bindet, aber von diesem nicht gespalten wird.

5. Verfahren gemäß Anspruch 1, bei dem ein modifiziertes Enzym verwendet wird und das modifizierte Enzym eine modifizierte Protease ist, in der die Peptidsequenz für das aktive Zentrum der genannten Protease derart verändert wurde, dass diese die Tag-Sequenz bindet, aber nicht spaltet.

6. Verfahren gemäß Anspruch 5, bei dem das modifizierte Enzym eine Serin-Protease ist, in der das aktive Zentrum des Serin-Rests durch eine nichtaktive Aminosäure wie Alanin ersetzt wurde.

7. Verfahren gemäß Anspruch 1, bei dem das Einfang-Protein eine modifizierte Enterokinase ist.

8. Verfahren gemäß Anspruch 1, wobei das modifizierte Enzym ein modifiziertes psychrophiles Enzym ist und die Bindung der Tag-Sequenz zum Einfang-Protein unterhalb der Denaturierungstemperatur des Enzyms stattfindet und die Eluation mittels Erhöhen der Temperatur nahe an die oder oberhalb der Denaturierungstemperatur des Enzyms durchgeführt wird.

9. Ein Verfahren zur Reinigung oder Isolierung eines rekombinanten Fusionspeptids, wobei das genannte Verfahren die folgenden Schritte umfasst:
e) Bildung eines Fusionspeptids, umfassend eine Tag-Peptidsequenz, die kovalent an eine Polypeptidsequenz gebunden ist, wobei die genannte Tag-Peptidsequenz eine Sequenz enthält, die aus der aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 und SEQ ID NO: 13 bestehenden Gruppe ausgewählt ist;
f) Kontaktieren des Fusionspeptids mit einem Enzym oder einem modifizierten Enzym, welches spezifisch an die Tag-Peptidsequenz bindet, um einen Komplex zwischen dem Enzym oder dem modifizierten Enzym und dem Fusionspeptid zu bilden;
g) Eluieren von nicht-komplexierten Peptiden, um die nicht-komplexierten Peptide von den komplexierten Peptiden zu trennen; und
h) Durchführen einer zweiten Eluation, in der das Fusionspeptid von dem Enzym oder dem modifizierten Enzym dissoziert wird,
wobei das genannte Verfahren nicht verlangt, dass die Tag-Sequenz ein Lysin oder Arginin an ihrem Carboxyl-Terminus aufweist, um die Bindung aufrecht zu erhalten

10. Ein Verfahren zum Markieren eines rekombinanten Peptids, bei dem das genannte Verfahren den Schritt der Bildung eines Fusionspeptids, umfassend eine Tag-Peptidsequenz, die kovalent an eine Polypeptidsequenz gebunden ist, umfasst und die genannte Tag-Peptidsequenz eine Peptidsequenz enthält, die aus der aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 und SEQ ID NO: 13 bestehenden Gruppe ausgewählt ist.

## Revendications

1. Procédé pour purifier ou isoler un peptide de fusion recombiné, ledit procédé comprenant les étapes consistant à :
a) former un peptide de fusion comprenant une séquence peptidique marqueuse liée de façon covalente à une séquence polypeptidique;
b) mettre le peptide de fusion en contact avec une enzyme ou une enzyme modifiée, qui se lie spécifiquement à la séquence peptidique marqueuse afin de former un complexe entre l'enzyme ou l'enzyme modifiée et le peptide de fusion;
c) éluer les peptides non complexés afin de séparer les peptides non complexés du peptide complexé; et
d) effectuer une deuxième élution dans laquelle le peptide de fusion est dissocié de l'enzyme ou de l'enzyme modifié;
dans lequel ledit procédé ne nécessite pas que la séquence marqueuse ait une lysine ou arginine au niveau de sa terminaison carboxyle pour maintenir la liaison.

2. Procédé selon la revendication 1, dans lequel la séquence marqueuse est un substrat ou un inhibiteur de l'enzyme.

3. Procédé selon la revendication 1, dans lequel l'enzyme ou l'enzyme modifiée est liée à un substrat solide.

4. Procédé selon la revendication 1, dans lequel la séquence marqueuse lie l'enzyme mais n'est pas clivée par celle-ci.

5. Procédé selon la revendication 1, dans lequel une enzyme modifiée est employée, et l'enzyme modifiée est une protéase modifiée dans laquelle la séquence peptidique pour le site actif de ladite protéase a été modifiée de sorte qu'elle lie, mais ne clive pas, la séquence marqueuse.

6. Procédé selon la revendication 5, dans lequel l'enzyme modifiée est une sérine protéase dans laquelle le résidu sérine du site actif a été remplacé par un acide aminé non actif tel que l'alanine.

7. Procédé selon la revendication 1, dans lequel la protéine de capture est une entérokinase modifiée.

8. Procédé selon la revendication 1, dans lequel l'enzyme modifiée est une enzyme psychrophile modifiée et la fixation des séquences marqueuses à la protéine de capture a lieu au-dessous de la température de dénaturation de l'enzyme et l'élution se réalise en élevant la température à proximité ou au-dessus de la température de dénaturation de l'enzyme.

9. Procédé pour purifier ou isoler un peptide de fusion recombiné, ledit procédé comprenant les étapes consistant à :
a) former un peptide de fusion comprenant une séquence peptidique marqueuse fixée de façon covalente à une séquence polypeptidique, ladite séquence peptidique marqueuse contenant une séquence choisie dans le groupe formé par SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, et SEQ ID NO:13;
b) mettre le peptide de fusion en contact avec une enzyme ou une enzyme modifiée qui se lie spécifiquement à la séquence peptidique marqueuse en formant un complexe entre l'enzyme ou l'enzyme modifiée et le peptide de fusion;
c) éluer les peptides non complexés afin de séparer les peptides non complexés du peptide complexé; et
d) effectuer une deuxième élution dans laquelle le peptide de fusion est dissocié de l'enzyme ou de l'enzyme modifiée;
dans lequel ledit procédé ne nécessite pas que la séquence marqueuse ait une lysine ou arginine au niveau de sa terminaison carboxyle pour maintenir la liaison.

10. Procédé d'étiquetage d'un peptide recombiné, ledit procédé comprenant l'étape de formation d'un peptide de fusion comprenant une séquence peptidique marqueuse fixée de façon covalente à une séquence polypeptidique, ladite séquence peptidique marqueuse contenant une séquence peptidique choisie dans le groupe formé par SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, et SEQ ID NO:13.
